(19) Europäisches Patentamt
European Patent Office
Office européen des brevets

(11) Publication number: 0 453 137 A2

# (12) EUROPEAN PATENT APPLICATION

(21) Application number: 91303007.8

(22) Date of filing: 05.04.91

(51) Int. Cl.[5]: **C07D 239/42**, C07D 409/12, C07D 405/12, A01N 43/54

(30) Priority: 14.04.90 JP 98466/90
30.11.90 JP 334470/90

(43) Date of publication of application: 23.10.91 Bulletin 91/43

(84) Designated Contracting States: CH DE ES FR GB IT LI

(71) Applicant: NIHON NOHYAKU CO., LTD.
1-2-5, Nihonbashi
Chuo-ku Tokyo (JP)

(72) Inventor: Tohnishi, Masanori
7-20, Nigawa Yurinocho
Nishinomiya-shi (JP)
Inventor: Kanaoka, Atsushi
3-24-110, Nankadai-3-chome
Kawachinagano-shi (JP)
Inventor: Endo, Katsutoshi
5-6, Hondacho
Kawachinagano-shi (JP)
Inventor: Nishimatsu, Tetsuyoshi
3-24-502, Nankadai-3-chome
Kawachinagano-shi (JP)
Inventor: Kanno, Hideo
2-2-708, Shirakawa-3-chome
Ibaraki-shi (JP)

(74) Representative: Votier, Sidney David et al
CARPMAELS & RANSFORD 43, Bloomsbury Square
London WC1A 2RA (GB)

## (54) Aminopyrimidine derivatives, a process for production thereof, and uses thereof.

(57) An aminopyrimidine derivative represented by the general formula (I):

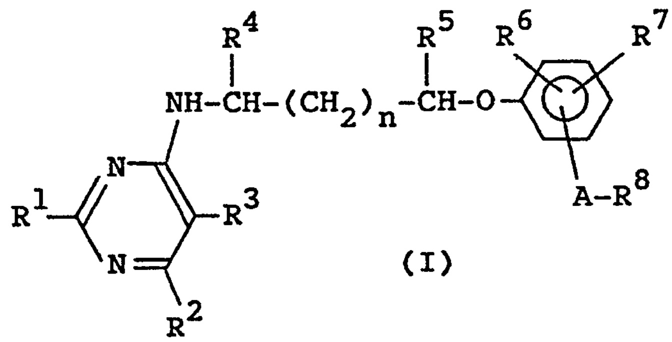

(I)

(wherein the symbols of substituents and the other symbols have the same meanings as those defined in the main text of the specification), a process for producing said derivative, and utilization of said derivative as an insecticidal, acaricidal and/or fungicidal composition.

EP 0 453 137 A2

BACKGROUND OF THE INVENTION

Field of the invention

The present invention relates to novel aminopyrimidine derivatives, a process for production thereof, and insecticidal, acaricidal and/or fungicidal compositions comprising said derivatives.

Related Art

Jap. Pat. Appln. Kokai (Laid-Open) Nos. SHO 57 (1982)-126481, SHO 59 (1984)-36666 and HEI 2 (1990)-196774 disclose pyrimidine derivatives. For improving their activity, the present inventors earnestly investigated and consequently found that the aminopyrimidine derivatives of the general formula (I) shown hereinafter of the present invention are novel compounds which have not been known in any literature, and that they have not only insecticidal and/or acaricidal activity higher than that of the well-known compounds disclosed in the above references but also fungicidal activity, whereby the present invention was accomplished.

DETAILED DESCRIPTION OF PREFERRED EMBODIMENTS

The present invention provides aminopyrimidine derivatives represented by the general formula (I):

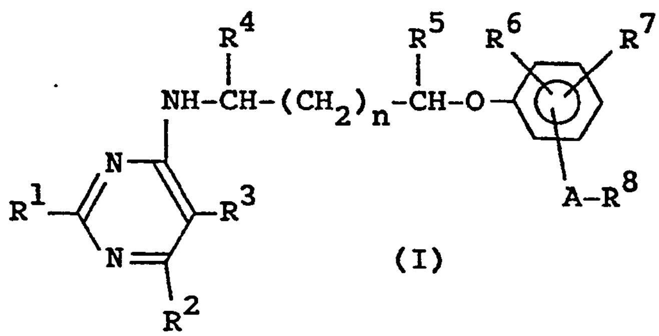

(I)

wherein $R^1$ is a hydrogen atom or a lower alkyl group, $R^2$ and $R^3$, which may be the same or different, are halogen atoms, lower alkyl groups, lower haloalkyl groups, lower alkenyl groups, lower alkynyl groups, cycloalkyl groups, lower alkoxy groups, lower haloalkoxy groups, lower alkylthio groups, or lower haloalkylthio groups, $R^4$ and $R^5$, which may be the same or different, are hydrogen atoms or lower alkyl groups, $R^6$ and $R^7$, which may be the same or different, are hydrogen atoms, halogen atoms, lower alkyl groups, lower haloalkyl groups, lower alkoxy groups, or lower alkylthio groups, A is $-(C(R^9)(R^{10}))_m-$ (wherein $R^9$ and $R^{10}$, which may be the same or different, are hydrogen atoms, lower alkyl groups, or lower haloalkyl groups, and m is an integer of 1 to 4), $R^8$ is a phenyl group which may have 1 to 4 substituents which may be the same or different and are selected from the group consisting of halogen atoms, nitro group, cyano group, lower alkyl groups, lower haloalkyl groups, lower alkoxy groups, lower haloalkoxy groups, lower alkylthio groups, lower haloalkylthio groups, unsubstituted amino group, and substituted amino group having as the substituent(s) one or two lower alkyl groups or acyl groups, which may be the same or different, or $R^8$ is an aromatic fused ring, a naphthyl group, or an oxygen- or sulfur-containing heterocyclic ring which may have 1 to 3 substituents selected from the group consisting of halogen atoms, nitro group, cyano group, lower alkyl groups, lower haloalkyl groups, lower alkoxy groups, lower haloalkoxy groups, lower alkylthio groups, and lower haloalkylthio groups, and n is zero or an integer of 1 to 3.

Compounds preferable as the aminopyrimidine derivatives are those in which $R^1$ is a hydrogen atom, $R^2$ is lower alkyl groups, $R^3$ is a halogen atom, each of $R^4$ and $R^5$ is a hydrogen atom, $R^6$ is a hydrogen atom, $R^7$ is a hydrogen atom, a halogen atom or a methyl group, and $R^8$ is an unsubstituted phenyl group or a substituted phenyl group having as the substituent(s) at least one member selected from the group consisting of halogen atoms, nitro group, cyano group, lower alkyl groups having 1 to 4 carbon atoms, lower alkoxy groups having 1 to 3 carbon atoms, and trifluoromethyl group.

Of these compounds, compounds in which n is zero and m is one are suitable.

The aminopyrimidine derivative of the general formula (I) of the present invention can be produced, for example, by the process illustrated below.

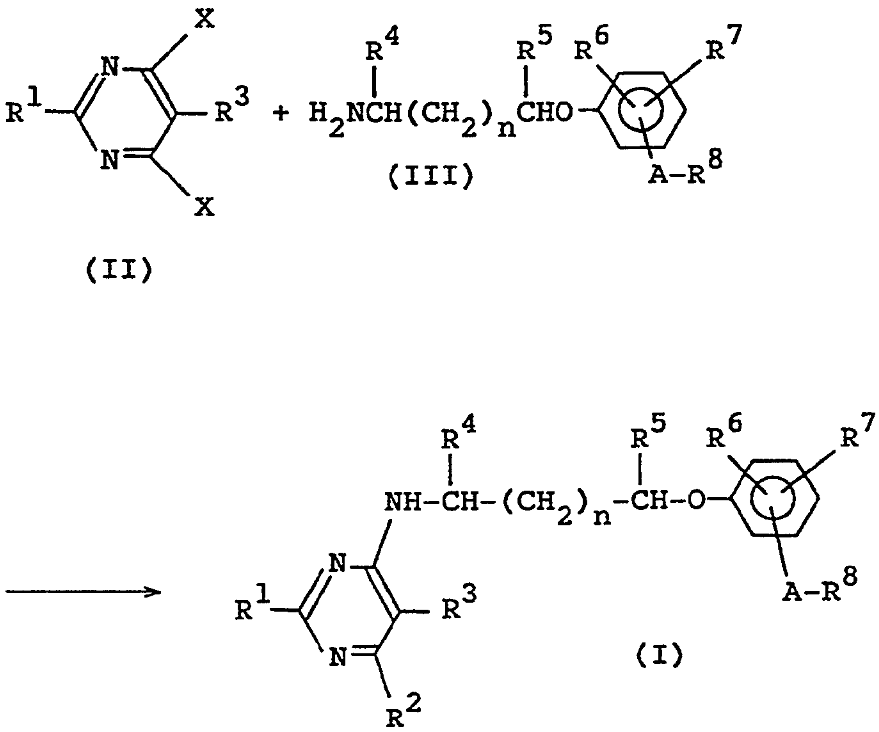

wherein $R^1$, $R^2$, $R^3$, $R^4$, $R^5$, $R^6$, $R^7$, $R^8$, A and n have the same meanings as those defined above, and X is a halogen atom.

That is, the aminopyrimidine derivative of the general formula (I) can be produced by reacting a halopyrimidine of the general formula (II) with an amine of the general formula (III) in the presence of a base and in the presence or absence of an inert solvent.

In the present specification, the term "lower" is used for expressing a number of carbon atoms of 1 to 6 for alkyl groups and 2 to 6 for alkenyl groups and alkynyl groups. In addition, it is used for expressing a number of carbon atoms of 3 to 6 for cycloalkyl groups.

The term "halogen" or "halo" generally means fluorine, chlorine, bromine or iodine, and for example, the term "lower haloalkyl" means a substituted alkyl group having 1 to 6 carbon atoms which has as the substituent(s) at least one of the above elements.

In the above reaction, as the inert solvent, anyone can be used so long as it does not inhibit the progress of the reaction greatly. There can be exemplified alcohols such as methanol, ethanol, propanol, ethylene glycol, etc.; halogenated hydrocarbons such as methylene chloride, chloroform, carbon tetrachloride, chlorobenzene, dichlorobenzene, etc.; aromatic hydrocarbons such as benzene, toluene, xylene, methylnaphthalene, etc.; esters such as ethyl acetate, etc.; chain ethers such as methyl Cellosolve, diethyl ether, ethylene glycol dimethyl ether, etc.; cyclic ethers such as dioxane, tetrahydrofuran, etc.; nitriles such as acetonitrile, benzonitrile, etc.; ketones such as acetone, methyl ethyl ketone, etc.; N,N-dimethylformamide; N,N-dimethylacetamide; dimethyl sulfoxide; and water. These inert solvents may be used singly or as a mixture thereof. The inert solvent used in the present invention is not limited to these solvents.

As the base usable in the present invention, an inorganic base or an organic base can be used. The inorganic base includes, for example, sodium carbonate, potassium carbonate, sodium hydroxide and potassium hydroxide. The organic base includes, for example, triethylamine, pyridine, N,N-dimethylaniline, N,N-dimethylaminopyridine and 1,8-diazabicyclo[5,4,0]-7-undecene (DBU).

The amount of the base used may be properly chosen in the range of 1/2 mole to excess moles per mole of the halopyrimidine of the general formula (II). It is preferably equimolar with or larger than the amount of the halopyrimidine.

When a combination of water and a water-insoluble organic solvent is used as the inert solvent, it is also possible to use a trialkylbenzylammonium halide such as trimethylbenzylammonium chloride, triethylbenzylammonium chloride or the like as the base which serves as a phase transfer catalyst.

Since the above reaction is an equimolar reaction, it is sufficient that the amine of the general formula (III) is used in an amount equimolar with the amount of the halopyridine of the general formula (II), though it is also possible to use the amine in excess.

In addition, the amine of the general formula (III) may be used in excess to serve also as the base.

The temperature for the above reaction may be chosen in the range of room temperature to the boiling range of the inert solvent used. The reaction is carried out preferably with heating.

Although the reaction time is varied depending on the reaction temperature, the degree of reaction, etc., it may be properly chosen in the range of several minutes to 48 hours.

After completion of the reaction, the desired compound is isolated from the reaction mixture containing the compound by a conventional method, and if necessary, purified by recrystallization, column chromatography, etc., whereby the aminopyrimidine derivative of the general formula (I) can be produced.

The halopyrimidine of the general formula (II) which is a starting compound for producing the aminopyrimidine derivative of the general formula (I) of the present invention, can be produced by any of the conventional processes described in references, for example, J. Med. Chem., 1968 (II), 608, and Jap. Pat. Appln. Kokai (Laid-Open) Nos. SHO 57 (1982)-126481 and SHO 62 (1987)-67.

The amine of the general formula (III) can be produced by a conventional production process, for example, the process illustrated below.

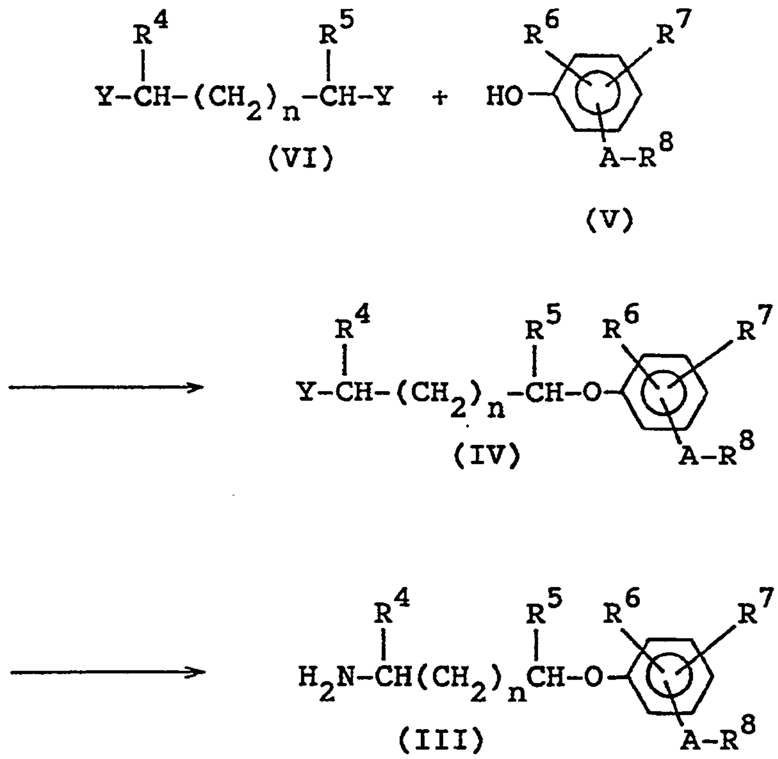

(VI) (V)

(IV)

(III)

wherein $R^4$, $R^5$, $R^6$, $R^7$, $R^8$, A and n have the same meanings as those defined above, and Y's, which may be the same or different, are halogen atoms.

That is, the amine of the general formula (III) can be produced by reacting a haloalkylene compound of the general formula (VI) with a phenol of the general formula (V) to obtain a compound of the general formula (IV), and aminating this compound (IV).

Typical compounds as the aminopyrimidine derivative of the general formula (I) of the present invention are listed in Table 1.

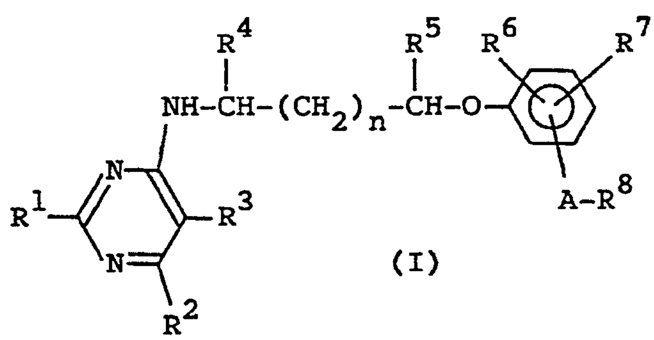

(I)

Table 1 (Compounds of the above formula (I) in which $R^1$=H, $R^2$=$CH_2CH_3$, $R^3$=Cl, $R^4$=H, $R^5$=H, and n=0.)

| No. | $R^6$ | $R^7$ | $R^8$ | (bonding position) | Physical properties |
|---|---|---|---|---|---|
| 1 | H | H | 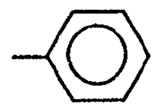 | $-CH_2-$ (3) | mp. 68.5-71.5°C |
| 2 | H | H | 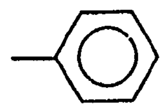 | $-CH_2-$ (4) | mp. 84-86°C |
| 3 | H | H | 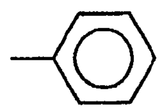 | 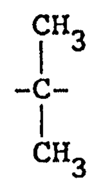 (4) | nD 1.5928 (24.7°C) |
| 4 | H | H | 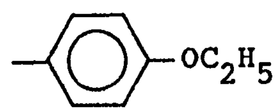 | $-CH_2-$ (4) | mp. 69-71°C |
| 5 | H | H | 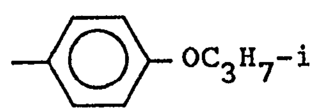 | $-CH_2-$ (4) | mp. 82-84°C |

- cont'd -

Table 1 (cont'd)

| | | | | | | |
|---|---|---|---|---|---|---|
| 6 | H | H | 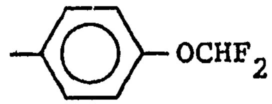 | $-CH_2-$ | (4) | mp. 80-81°C |
| 7 | H | 2-Cl | 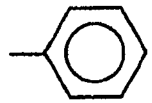 | $-CH_2-$ | (4) | mp. 64-65°C |
| 8 | H | 2-Br | 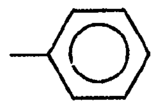 | $-CH_2-$ | (4) | mp. 80-82°C |
| 9 | H | 2-F | 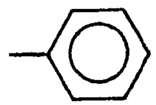 | $-CH_2-$ | (4) | mp. 63-64°C |
| 10 | H | $2-NO_2$ | 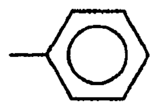 | $-CH_2-$ | (4) | mp. 55-57°C |
| 11 | H | $2-CH_3$ | 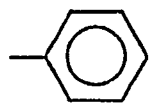 | $-CH_2-$ | (4) | mp. 46-47°C |
| 12 | H | $3-CH_3$ | 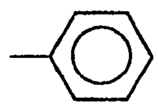 | $-CH_2-$ | (4) | mp. 108-110°C |

- cont'd -

Table 1 (cont'd)

| | | | | | |
|---|---|---|---|---|---|
| 13 | H | 2-$CH_3$ | 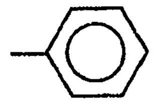 | $-(CH_2)_2-$ (4) | nD 1.5892 (20.7°C) |
| 14 | H | 2-$CH_3$ | 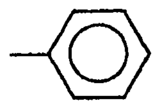 | $-(CH_2)_3-$ (4) | nD 1.5830 (20.5°C) |
| 15 | H | 2-$CH_3$ | 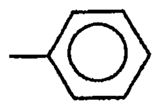 | $-CH_2CH(CH_3)-$ (4) | nD 1.5822 (24.5°C) |
| 16 | H | 2-$C_2H_5$ | 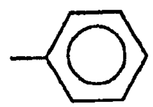 | $-CH_2-$ (4) | mp. 49-50°C |
| 17 | H | 2-$CH_3$ | 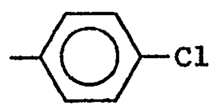 | $-CH_2-$ (4) | mp. 68-70°C |
| 18 | H | 2-$CH_3$ | 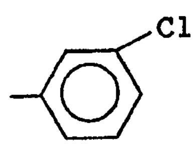 | $-CH_2-$ (4) | mp. 66-67°C |

- cont'd -

Table 1 (cont'd)

| | | | | | | |
|---|---|---|---|---|---|---|
| 19 | H | 2-$CH_3$ | 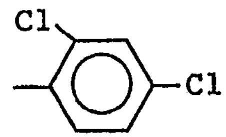 | -$CH_2$- | (4) | mp. 90-92°C |
| 20 | H | 2-$CH_3$ | 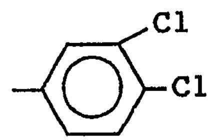 | -$CH_2$- | (4) | mp. 85-87°C |
| 21 | H | 2-$CH_3$ | 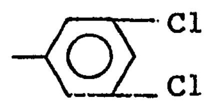 | -$CH_2$- | (4) | mp. 68-71°C |
| 22 | H | 2-$CH_3$ | 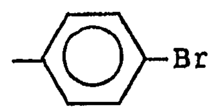 | -$CH_2$- | (4) | nD 1.6069 (20.3°C) |
| 23 | H | 2-$CH_3$ | 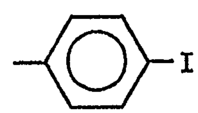 | -$CH_2$- | (4) | nD 1.6192 (24.8°C) |
| 24 | H | 2-$CH_3$ | 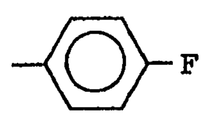 | -$CH_2$- | (4) | nD 1.5835 (20.1°C) |

- cont'd -

Table 1 (cont'd)

| | | | | | | |
|---|---|---|---|---|---|---|
| 25 | H | 2-$CH_3$ | 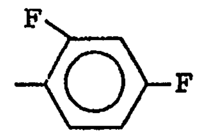 | $-CH_2-$ | (4) | mp. 51-52°C |
| 26 | H | 2-$CH_3$ | 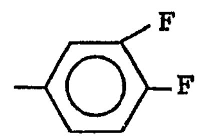 | $-CH_2-$ | (4) | nD 1.5761 (24.4°C) |
| 27 | H | 2-$CH_3$ | 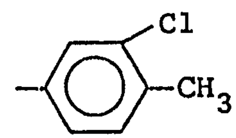 | $-CH_2-$ | (4) | mp. 81-83°C |
| 28 | H | 2-$CH_3$ | 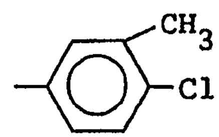 | $-CH_2-$ | (4) | mp. 75-77°C |
| 29 | H | 2-$CH_3$ | 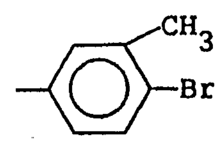 | $-CH_2-$ | (4) | mp. 95-97°C |

- cont'd -

Table 1 (cont'd)

| | | | | | | |
|---|---|---|---|---|---|---|
| 30 | H | 2-$CH_3$ | 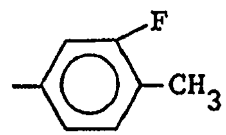 | $-CH_2-$ | (4) | mp. 48-50°C |
| 31 | H | 2-$CH_3$ | 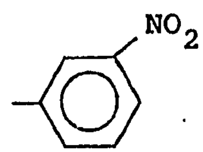 | $-CH_2-$ | (4) | mp. 90-91°C |
| 32 | H | 2-$CH_3$ | 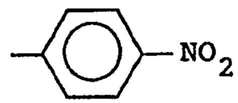 | $-CH_2-$ | (4) | mp. 84-86°C |
| 33 | H | 2-$CH_3$ | 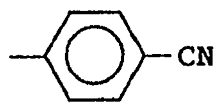 | $-CH_2-$ | (4) | mp. 71-73°C |
| 34 | H | 2-$CH_3$ | 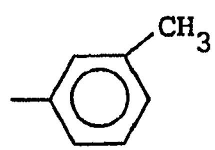 | $-CH_2-$ | (4) | nD 1.5920 (19.9°C) |
| 35 | H | 2-$CH_3$ | 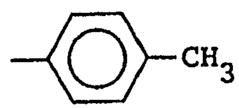 | $-CH_2-$ | (4) | mp. 59-61°C |

- cont'd -

Table 1 (cont'd)

| | | | | | | |
|---|---|---|---|---|---|---|
| 36 | H | 2-$CH_3$ | 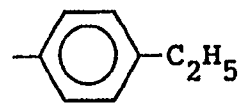 | $-CH_2-$ | (4) | mp. 65-67°C |
| 37 | H | 2-$CH_3$ | 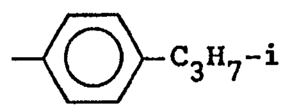 | $-CH_2-$ | (4) | nD 1.5808 (20.5°C) |
| 38 | H | 2-$CH_3$ | 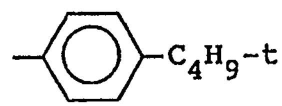 | $-CH_2-$ | (4) | nD 1.7061 (20.3°C) |
| 39 | H | 2-$CH_3$ | 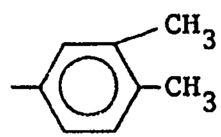 | $-CH_2-$ | (4) | mp. 65-67°C |
| 40 | H | 2-$CH_3$ | 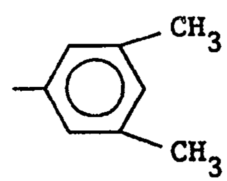 | $-CH_2-$ | (4) | nD 1.5878 (20.7°C) |
| 41 | H | 2-$CH_3$ | 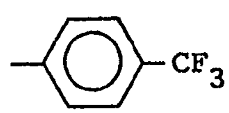 | $-CH_2-$ | (4) | nD 1.5601 (20.4°C) |

- cont'd -

Table 1 (cont'd)

| | | | | | | |
|---|---|---|---|---|---|---|
| 42 | H | 2-$CH_3$ | 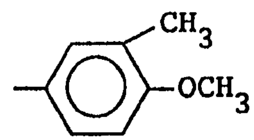 | $-CH_2-$ | (4) | nD 1.5888 (24.6°C) |
| 43 | H | 2-$CH_3$ | 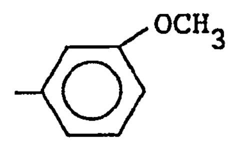 | $-CH_2-$ | (4) | nD 1.5933 (24.8°C) |
| 44 | H | 2-$CH_3$ | 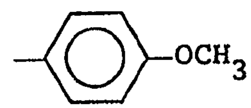 | $-CH_2-$ | (4) | mp. 51-52°C |
| 45 | H | 2-$CH_3$ | 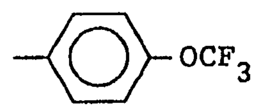 | $-CH_2-$ | (4) | nD 1.5526 (24.5°C) |
| 46 | H | 2-$CH_3$ | 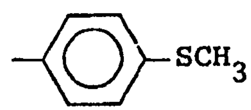 | $-CH_2-$ | (4) | mp. 87-89°C |
| 47 | H | 2-$CH_3$ | 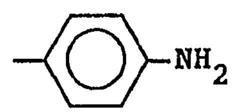 | $-CH_2-$ | (4) | mp. 129-131°C |

- cont'd -

Table 1 (cont'd)

| | | | | | |
|---|---|---|---|---|---|
| 48 | H | 2-$CH_3$ | 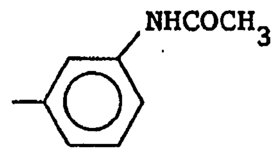 | $-CH_2-$ (4) | mp. 135-137°C |
| 49 | H | 2-$CH_3$ | 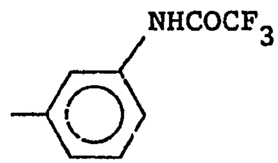 | $-CH_2-$ (4) | mp. 140-142°C |
| 50 | H | 2-$CH_3$ | 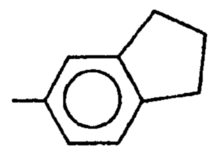 | $-CH_2-$ (4) | nD 1.5906 (24.6°C) |
| 51 | H | 2-$CH_3$ | 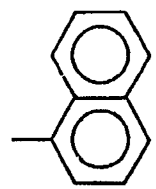 | $-CH_2-$ (4) | nD 1.6193 (20.4°C) |
| 52 | H | 2-$CH_3$ | 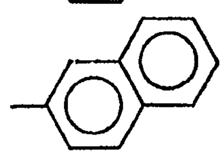 | $-CH_2-$ (4) | mp. 65-66°C |

- cont'd -

Table 1 (cont'd)

| | | | | | | |
|---|---|---|---|---|---|---|
| 53 | H | 2-$CH_3$ | 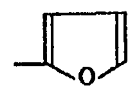 | $-CH_2-$ | (4) | nD 1.5835 (24.4°C) |
| 54 | H | 2-$CH_3$ | 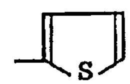 | $-CH_2-$ | (4) | nD 1.6009 (24.5°C) |
| 55 | 2-$CH_3$ | 3-$CH_3$ | 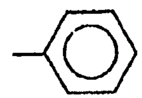 | $-CH_2-$ | (4) | mp. 84-86°C |
| 56 | 2-$CH_3$ | 5-$CH_3$ | 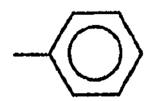 | $-CH_2-$ | (4) | mp. 97-99°C |
| 57 | 2-$CH_3$ | 6-$CH_3$ | 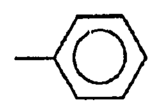 | $-CH_2-$ | (4) | mp. 74-76°C |
| 58 | 2-$CH_3$ | 6-Cl | 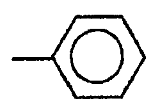 | $-CH_2-$ | (4) | nD 1.5931 (25.1°C) |

Typical examples of the present invention are given below.

Example 1

4-[2-(4-Benzylphenoxy)ethylamino]-5-chloro-6-ethylpyrimidine (compound No. 2)

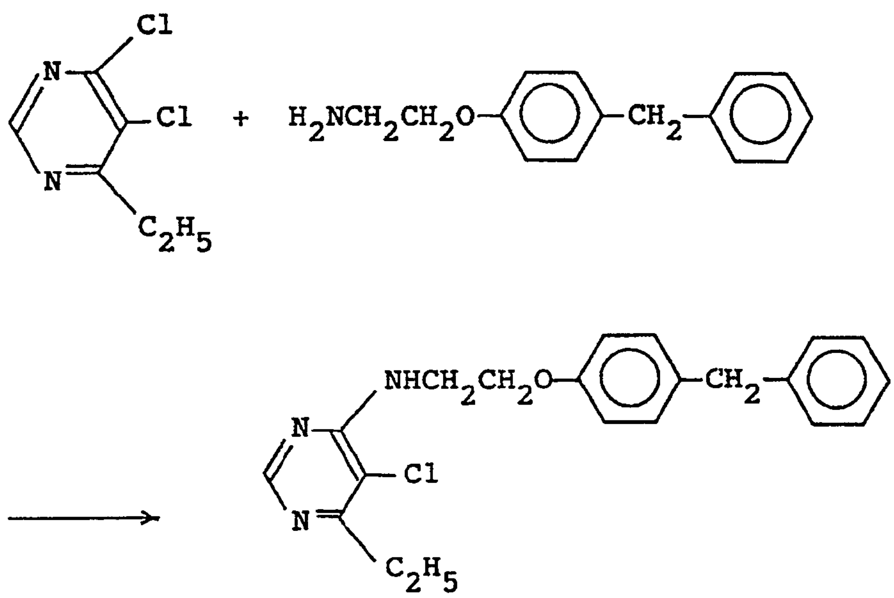

In 20 ml of toluene were dissolved 0.35 g (0.002 mole) of 4,5-dichloro-6-ethylpyrimidine and 0.45 g (0.002 mole) of 2-(4-benzylphenoxy)ethylamine, followed by adding thereto 0.24 g (0.0024 mole) of triethylamine, and the reaction was carried out with heating and refluxing for 3 hours.

After completion of the reaction, the reaction mixture was poured into cold water, and the organic layer thus separated was washed with water and dried over anhydrous magnesium sulfate. Then, the toluene was distilled off under reduced pressure, and the resulting residue was purified by a column chromatography (ethyl acetate : n-hexane = 1 : 2) to obtain 0.39 g of the desired compound as white crystals.

Physical properties: mp. 84 - 86°C, yield 53%.

Example 2

4-[2-(4-Benzyl-2-methylphenoxy)ethylamino]-5-chloro-6-ethylpyrimidine (compound No. 11)

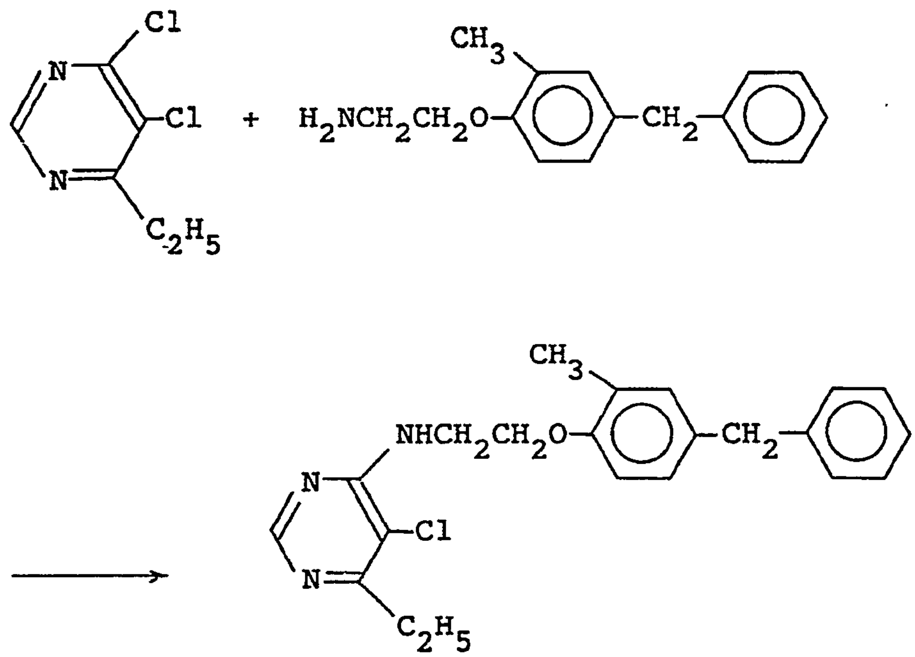

In 20 ml of dimethylformamide were dissolved 0.35 g (0.002 mole) of 4,5-dichloro-6-ethylpyrimidine and 0.48 g (0.002 mole) of 2-(4-benzyl-2-methylphenoxy)-ethylamine, followed by adding thereto 0.21 g (0.0015 mole) of anhydrous potassium carbonate, and the reaction was carried out with heating and stirring at 110°C for 3 hours.

After completion of the reaction, the reaction mixture was poured into cold water, and the oily substance thus separated was extracted with diethyl ether. The extracted solution was washed with water and dried over anhydrous magnesium sulfate, and the solvent was distilled off under reduced pressure. The oily substance thus obtained was purified by a column chromatography (ethyl acetate : n-hexane = 1 : 2) obtain 0.58 g of the desired compound as white crystals.

Physical properties: mp. 46 - 47°C, yield 76%.

The aminopyrimidine derivative of the general formula (I) of the present invention has an excellent insecticidal and/or acaricidal effect on various agricultural and horticultural insect pests, for example, Hemiptera such as planthoppers (Delphacidae [e.g. small brown planthopper (Laodelphax striatellus), brown rice planthopper (Nilaparvata lugens), and white-backed rice planthopper (Sogatella furcifera)] and leafhoppers (Deltocephalidae) [e.g. green rice leafhopper (Nephotettix cincticeps) and aster leafhopper (Macrosteres fascifrons)], Lepidoptera including diamondback moth (Plutella xylostella), rice stem borer (Chilo suppressalis), rice leafroller (Cnaphalocrocis medinalis), common cutworm (Spodoptera litura), etc., and Coleoptera including twenty-eight-spotted ladybird (Epilachna vigintioctopunctata), red flour beetle (Tribolium castaneum), etc.; acarines such as citrus red mite (Panonychus citri), two-spotted spider mite (Tetranychus urticae), etc.; and sanitary insect pests such as mosquito, muscid flies, etc.

Moreover, the aminopyrimidine derivative of the general formula (I) of the present invention is useful also as an agricultural and horticultural fungicide and has an excellent fungicidal activity, for example, against rice blast (Piricularia oryzae), powder mildew (Erysiphe graminis), downy mildew (Pseudoperonospora cubensis), and gray mold (Botrytis cinerea).

Therefore, the aminopyridine derivative of the general formula (I) of the present invention is useful for controlling various diseases and insect pests by application to plants, cereals and the like, which are to be protected against various insect pests, acarines and diseases.

For example, the aminopyrimidine derivative of the general formula (I) of the present invention is useful for protecting cereals such as rice, barley, wheat, oats rye, corn, etc., vegetables, flower and ornamental plants, trees. cotton, fruit trees, timbers, harvested cereals, pasture grass, lawn, wood products, etc. against diseases and insect pests.

The aminopyrimidine derivative of the general formula (I) of the present invention, when applied as an insecticidal, acaricidal and/or fungicidal composition, is generally made up, according to the customary procedure for preparing agricultural chemicals, into a form convenient for use.

In detail, the aminopyrimidine derivative of the general formula (I) of the present invention is blended with a suitable inert carrier and if necessary, further with an adjuvant, in proper ratios, and the mixture is made up

into a suitable form of preparation, e.g. a suspension, emulsifiable concentrate, solution, wettable powder, granules, dusts, or tablets, through dissolution, dispersion, suspension, mixing, impregnation, adsorption, or sticking.

In the present invention, either solid or liquid inert carriers may be used. Suitable materials as solid carriers include soybean fluor, cereal flour, wood flour, bark flour, saw dust, powdered tobacco stalks, powdered walnut shells, bran, powdered cellulose, extraction residues of vegetables, powdered synthetic polymers or resins, clays (e.g. kaolin, bentonite, and acid clay), talcs (e.g. talc and pyrophyllite), silica powders or flakes [e.g. diatomaceous earth, silica sand, mica and white carbon (i.e. highly dispersed synthetic silicic acid, also called finely divided hydrated silica or hydrated silicic acid, and some of commercially available products contain calcium silicate as the major component)], activated carbon, powdered sulfur, powdered pumice, calcined diatomaceous earth, ground bricks, fly ash, sand, calcium carbonate powder, calcium phosphate powder, and other inorganic or mineral powders, chemical fertilizers (e.g. ammonium sulfate, ammonium phosphate, ammonium nitrate, urea, and ammonium chloride) and compost. These materials may be used alone or as a mixture thereof.

Suitable materials as liquid carriers include liquids which themselves show some solution-forming activity as well as liquids which do not show any solution-forming activity but can disperse active ingredients with the aid of adjuvants. Typical examples of such liquids, which may be used alone or as a mixture thereof, are water, alcohols (including methanol, ethanol, isopropanol, butanol, and ethylene glycol), ketones (including acetone, methyl ethyl ketone, methyl isobutyl ketone, diisobutyl ketone, and cyclohexanone), ethers (including ethyl ether, dioxane, cellosolve, dipropyl ether, and tetrahydrofuran), aliphatic hydrocarbons (including gasoline, and mineral oils), aromatic hydrocarbons (including benzene, toluene, xylene, solvent naphtha, and alkyl naphthalenes), halogenated hydrocarbons (including dichloroethane, chloroform, carbon tetrachloride, and chlorinated benzene), esters (including ethyl acetate, diisopropyl phthalate, dibuthl phthalate, and dioctyl phthalate), amides (including dimethylformamide, diethylformamide, and dimethylacetamide), nitriles (including acetonitrile), and dimethyl sulfoxide.

Typical examples of the adjuvant are given below. They may be used depending on purposes singly or in combination of two or more thereof. No adjuvant is needed at all in some cases.

For the purpose of emulsifying, dispersing, solubilizing, and/or wetting active ingredients, there may be used surface active agents such as polyoxyethylene alkyl ethers, polyoxyethylene alkylaryl ethers, polyoxyethylene higher fatty acid esters, polyoxyethylene resinates, polyoxyethylene sorbitan monolaurate, polyoxyethylene sorbitan monooleate, alkylarylsulfonates, naphthalenesulfonic acid condensation products, ligninesulfonates, and higher alcohol sulfate esters.

For the purpose of stabilizing the dispersion of active ingredients, tackifying and/or binding them, there may be used adjuvants, for example, casein, gelatin, starch, methylcellulose, carboxymethylcellulose, gum arabic, polyvinyl alcohol, turpentine oil, bran oil, bentonite, and ligninsulfonates.

For the purpose of improving flow properties of solid products, there may be used adjuvants, for example, wax, stearates, and phosphoric acid alkyl esters.

Adjuvants, e.g. naphthalenesulfonic acid condensation products, and condensed phosphates, can be used as peptizers in dispersible products.

Adjuvants, e.g. silicone oils, can also be used as defoaming agents.

The content of active ingredients may be varied as occasion demands. For example, for preparing a powdered or granulated product, the content is suitably 0.01 to 50% by weight. Also for preparing an emulsifiable concentrate or wettable powder product, the content is suitably 0.01 to 50% by weight.

For controlling various diseases and insect pests, the insecticidal, acaricidal and/or fungicidal composition comprising the aminopyrimidine derivative of the general formula (I) of the present invention as active ingredient is applied in a disease- and/or insect pest-controlling dosage as such or after properly diluted with or suspended in water or other suitable medium, to the diseases and/or insect pests to be controlled or an area where the emergence or growth of the diseases and insect pests is undesirable.

Although the applying dosage of the insecticidal, acaricidal and/or fungicidal composition comprising the aminopyrimidine derivative of the general formula (I) of the present invention as active ingredient is varied depending on various factors such as the purpose of application, the objective diseases and/or insect pests, the growth state of crops, the emergence tendency of diseases and insect pests, weather, environmental conditions, the type of formulation, how, where and when said composition is applied, etc., it is properly chosen in the range of 0.1 g to 1 kg in terms of the active ingredient, per 10 ares, depending on purposes.

In order to expand both the spectrum of controllable diseases and/or insect pests and the period of time when effective applications are possible or to reduce the dosage, the insecticidal, acaricidal and/or fungicidal composition comprising the aminopyrimidine derivative of the general formula (I) of the present invention as active ingredient can be used in admixture with other insecticides, acaricides or fungicides, for example, the

following agents (the names in the parentheses after the chemical names are common names).

Insecticides and acaricides:

0,S-dimethyl acetylphosphoramidothioate (Acephate),
0,0-diethyl 0-5-phenylisoxazol-3-yl phosphorothioate (Isoxathion),
0,0-diethyl 0-3,5,6-trichloro-2-pyridyl phosphorothioate (Chlorpyrifos),
0,0-dimethyl 0-3,5,6-trichloro-2-pyridyl phosphorothioate (Chlorpyrifos-methyl),
0,0-dimethyl S-methylcarbamoylmethyl phosphorodithioate (Dimethoate),
0,0-diethyl 0-isopropyl-6-methylpyrimidinyl-4-yl phosphorothioate (Diazinon),
S-2-ethylthioethyl 0,0-dimethyl phosphorodithioate (Thiometon),
0-(1,6-dihydro-6-oxo-1-phenylpyridazin-3-yl) 0,0-diethyl phosphorothioate (Pyridaphenthion),
0-2-diethylamino-6-methylpyrimidin-4-yl 0,0-dimethyl phosphorothioate (Pirimifos-methyl),
4-(methylthio)phenyl dipropyl phosphate (Propaphos),
dimethyl (E)-1-methyl-2-(methylcarbamoyl)vinyl phosphate (Monocrotophos),
2,2-dichlorovinyl dimethyl phosphate (Dichlovos),
S-2,3-dihydro-5-methoxy-2-oxo-1,3,4-thiadiazol-3-ylmethyl 0,0-dimethyl phophorodithioate (Methidathion),
0-ethyl 0-4-nitrophenyl phenylphosphorothioate (EPN),
0,0-dimethyl 0-4-nitro-m-tolyl phosphorothioate (Fenitrothion),
0,0-dimethyl 0-4-methylthio-m-tolyl phosphorothioate (Fenthion),
ethyl 2-dimethoxythiophosphorylthio-2-phenyl-acetate (Phenthoate),
2-dimethylamino-5,6-dimethylpyrimidin-4-yl dimethylcarbamate (sirimicarb),
2-sec-butylphenyl methylcarbamate (BPMC),
2-isopropylphenyl methylcarbamate (Isoprocarb),
3,4-xylyl methylcarbamate (Xylylcarb),
m-tolyl methylcarbamate (Metolcarb),
1-naphthyl methylcarbamate (Carbaryl),
3,5-xylyl methylcarbamate (XMC),
2-(4-ethoxyphenyl)-2-methylpropyl 3-phenoxybenzyl ether (Ethofenprox),
1,3-di(carbamoylthio)-2-dimethylaminopropane hydrochloride (Cartap hydrochloride),
1-(4-chlorophenyl)-3-(2,6-difluorobenzoyl)urea (Diflubenzuron),
2-tert-butylimino-3-isopropyl-5-phenyl-3,4,5,6-tetrahydro-2H-1,3,5-thiadiazin-4-one (Buprofezin),
ethyl N-[2,3-dihydro-2,2-dimethylbenzofuran-7-yloxycarbonyl(methyl)aminothio]-N-isopropyl-β-alaninate (Benfuracarb),
S-methyl-N-(methylcarbamoyloxy)thioacetimidate (Methomyl),
ethyl 4,4′-dichlorobenzilate (Chlorobenzilate),
2,2,2-trichloro-1,1-bis(4-chlorophenyl)ethanol (dicofol),
isopropyl 4,4′-dibromobenzilate (Bromopropylate),

Fungicides:

diisopropyl 1,3-dithiolan-2-ylidenemalonate (Isoprothiolane),
3-(3,5-dichlorphenyl)-N-isopropyl-2,4-dioxoimidazolidine-1-carboxamide (Iprodione),
6-(3,5-dichlorophenyl-p-tolyl)pyridazin-3(2H)-one (Diclomezine),
dimethyl 4,4′-(o-phenylene)bis(3-thioallophanate) (Thiophanate-methyl),
5-methyl-1,2,5-triazolo[3,4-b][1,3]benzothiazole (tricyclazole),
3-hydroxy-5-methylisoxazole (Himexazol),
1,2,5,6-tetrahydropyrolo[3,2,1-ij]quinolin-4-one (pyroquilon),
3-(3,5-dichlorophenyl)-5-methyl-5-vinyl-1,3-oxazolidine-2,4-dione (vinclozolin),
4,5,6,7-tetrachlorophthalide (Phthalide),
α,α,α-trifluoro-3′-isopropoxy-o-toluanilide (Flutranil),
N-(3,5-dichlorophenyl)-1,3-dimethylcyclopropane-1,2-dicarboxyimide (Procymidone),
3-allyloxy-1,2-benzothiazole 1,1-dioxide (Probenazole),
methyl 1-(butylcarbamoyl)benzimidazol-2-ylcarbamate (Benomyl),
3′-isopropoxy-o-toluanilide (Mepronil),
0-ethyl S,S-diphenyl phosphorodithioate (Edifenphos),
S-benzyl 0,0-di-isopropyl phosphorothioate (Iprobenfos),
tetrachloroisophthalonitrile (Chlorothalonil),
Validamycin,
Blasticidin-S,
Polyoxins.

Typical test examples and formulation examples of the present invention are described below but they should not be construed as limiting the scope of the invention.

Formulation Example 1

| | |
|---|---|
| Each compound of the invention | 50 parts |
| Mixture of diatomaceous earth and clay | 45 parts |
| Polyoxyethylene nonylphenyl ether | 5 parts |

A wettable powder was prepared by mixing uniformly and grinding the above ingredients.

Formulation Example 2

| | |
|---|---|
| Each compound of the invention | 20 parts |
| Tetrahydrofuran | 65 parts |
| Mixture of polyoxyethylene nonylphenyl ether and alkylbenzene sulfonate | 15 parts |

An emulsifiable concentrate was prepared by mixing uniformly the above ingredients to effect dissolution.

Formulation Example 3

| | |
|---|---|
| Each compound of the invention | 4 parts |
| Mixture of diatomaceous earth, clay and talc | 95 parts |
| Calcium stearate | 1 part |

A dust was prepared by mixing uniformly and grinding the above ingredients.

Formulation Example 4

| | |
|---|---|
| Each compound of the invention | 3 parts |
| Mixed powder of bentonite and clay | 92 parts |
| Calcium ligninsulfonate | 5 parts |

Granules were prepared by mixing the above ingredients uniformly, and kneading the resulting mixture together with a suitable amount of water, followed by granulation and drying.

Test Example 1

Insecticidal effect on brown rice planthopper (Nilaparvata lugens)

Five rice seedlings at the 1.5 leaf stage were immersed in a 200 ppm liquid chemical containing each compound of the present invention as active ingredient for 30 seconds, air-dried, transferred to a glass tube containing 1 ml of watter, inoculated with third-instar larvae of brown rice planthopper, and allowed to stand in a room thermostated at 25°C.

Eight days after the treatment, the dead and alive were counted, and the mortality was calculated according to the following equation and the effect was judged according to the criterion shown below. The test was carried out with a single group in three replications.

$$\text{Mortality (\%)} = \left( \frac{\text{Number of dead larvae}}{\text{Number of inoculated larvae}} \right) \times 100$$

Criterion for the judgement

| Effect | Mortality (%) |
|---|---|
| A | 100 |
| B | 90 - 99 |
| C | 80 - 89 |
| D | 50 - 79 |

The results obtained are shown in Table 2.

Table 2

| Compound No. | Effect | Compound No. | Effect |
|---|---|---|---|
| 1 | A | 17 | A |
| 2 | A | 18 | A |
| 3 | B | 19 | A |
| 4 | A | 20 | A |
| 5 | A | 22 | A |
| 6 | A | 23 | A |
| 7 | A | 24 | A |
| 8 | A | 25 | A |
| 9 | A | 26 | A |
| 11 | A | 27 | A |
| 12 | B | 28 | A |
| 13 | A | 29 | A |
| 15 | A | 30 | A |
| 16 | A | 31 | A |

(to be continued)

Table 2 (continued)

| Compound No. | Effect | Compound No. | Effect |
|---|---|---|---|
| 32 | A | 45 | A |
| 33 | A | 46 | A |
| 34 | A | 47 | A |
| 35 | A | 48 | D |
| 36 | A | 49 | A |
| 37 | A | 50 | A |
| 38 | A | 51 | B |
| 40 | A | 52 | A |
| 41 | A | 55 | A |
| 42 | A | 56 | B |
| 43 | A | 57 | A |
| 44 | A | 58 | A |

Test Example 2

Insecticidal effect on common cutworm (Spodoptera litura)

A cabbage leaf disk with a diameter of 8 cm was immersed in a 200 ppm liquid chemical containing each compound of the present invention as active ingredient for 30 seconds, air-dried, placed in a Petri dish with a diameter of 9 cm, and inoculated with second-instar larvae of common cutworm.

Eight days after the treatment, the dead and alive were counted, and in the same manner as in Test Example 1, the mortality was calculated and the effect was judged. The results obtained are shown in Table 3.

Table 3

| Compound No. | Effect | Compound No. | Effect |
|---|---|---|---|
| 5 | A | 29 | D |
| 6 | C | 30 | A |
| 7 | A | 31 | D |
| 9 | A | 32 | C |
| 11 | A | 33 | A |
| 12 | C | 34 | A |
| 13 | A | 35 | A |
| 15 | B | 36 | A |
| 17 | C | 37 | A |
| 18 | C | 38 | A |
| 19 | A | 39 | A |
| 20 | A | 41 | A |
| 22 | A | 42 | A |
| 23 | A | 43 | A |
| 24 | A | 44 | C |
| 25 | A | 45 | A |
| 26 | A | 46 | A |
| 27 | A | 47 | A |
| 28 | A | 49 | A |

(to be continued)

Table 3 (continued)

| Compound No. | Effect | Compound No. | Effect |
|---|---|---|---|
| 50 | C | 55 | C |
| 53 | D | 57 | A |
| 54 | C | | |

Test Example 3

Insecticidal effect on two-spotted spider mite (Tetranychus urticae)

In a greenhouse, female adult two-spotted spider mites were released on a potted soybean plant. After 24 hours, the plant was treated with a 200 ppm liquid chemical containing each compound of the present invention as active ingredient by means of a spray gun.

Eight days after the chemical treatment, the dead and alive were counted, and in the same manner as in Test Example 1, the mortality was calculated and the effect was judged.

The results obtained are shown in Table 4.

Table 4

| Compound No. | Effect | Compound No. | Effect |
|---|---|---|---|
| 1 | B | 26 | A |
| 2 | B | 27 | A |
| 5 | B | 28 | A |
| 6 | C | 29 | A |
| 7 | A | 30 | A |
| 8 | A | 31 | A |
| 9 | A | 32 | A |
| 11 | A | 33 | A |
| 12 | A | 34 | A |
| 13 | A | 35 | A |
| 14 | A | 36 | A |
| 15 | A | 37 | A |
| 16 | A | 38 | A |
| 17 | B | 39 | A |
| 18 | A | 40 | A |
| 19 | B | 41 | A |
| 20 | B | 42 | A |
| 21 | A | 43 | B |
| 22 | A | 44 | B |
| 23 | A | 45 | A |
| 24 | A | 46 | A |
| 25 | A | 47 | A |

(to be continued)

Table 4 (continued)

| Compound No. | Effect | Compound No. | Effect |
|---|---|---|---|
| 48 | A | 53 | A |
| 49 | A | 54 | A |
| 50 | A | 55 | A |
| 51 | A | 56 | A |
| 52 | A | | |

Test Example 4

Insecticidal effect on twenty-eight-spotted ladybird (Epilachna vigintioctopunctata)

Leaflets of a tomato plant grown in a greenhouse were immersed in a 100 ppm liquid chemical containing each compound of the present invention as active ingredient for 30 seconds, and air-dried. Then, 1 ml of water was placed in a Petri dish whose bottom had been covered with a filter paper, and three of the treated tomato leaflets were placed in the Petri dish, inoculated with second-instar larvae of twenty-eight-spotted ladybird, and allowed to stand in a room thermostated at 25°C.

Eight days after the chemical treatment, the dead and alive were counted, and in the same manner as in Test Example 1, the mortality was calculated and the effect was judged.

The results obtained are shown in Table 5.

Table 5

| Compound No. | Effect | Compound No. | Effect |
|---|---|---|---|
| 10 | A | 34 | A |
| 18 | A | 35 | A |
| 22 | A | 36 | A |
| 24 | A | 39 | A |
| 26 | A | 42 | D |
| 27 | D | 43 | A |
| 28 | A | 44 | A |
| 29 | D | 46 | A |
| 30 | A | 50 | A |
| 32 | A | 53 | A |
| 33 | A | 54 | A |

Test Example 5

Controlling effect on downy mildew (Pseudoperonospora cubensis)

Potted cucumber plants of 2 leaf stage were sprayed with a 200 ppm liquid chemical containing each compound of the present invention as active ingredient. After 24 hours, they were inoculated with a suspension of zoospores of Pseudoperonospora cubensis by spraying.

After the inoculation, the plants were placed in a moist chamber at 25°C for 1 day and then a greenhouse for 6 days to cause the disease sufficiently. Thereafter, the percentage of lesion area of each leaf was measured, and the controlling degree was calculated by comparing the percentage of lesion area measured for a treated group with that measured for an untreated group. The effect was judged according to the criterion shown below.

$$\text{Controlling degree} = \left(1 - \frac{\text{Percentage of lesion area measured for treated group}}{\text{Percentage of lesion area measured for untreated group}}\right) \times 100$$

Criterion for the judgement

| Effect | Controlling degree (%) |
|---|---|
| A | 100 - 95 |
| B | 94 - 80 |
| C | 79 - 60 |
| D | 59 - 0 |

The results obtained are shown in Table 6.

Table 6

| Compound No. | Effect | Compound No. | Effect |
|---|---|---|---|
| 1 | C | 26 | A |
| 4 | B | 27 | A |
| 5 | B | 28 | A |
| 6 | A | 29 | A |
| 7 | B | 30 | A |
| 8 | C | 31 | A |
| 9 | B | 32 | A |
| 11 | A | 33 | A |
| 12 | B | 34 | A |
| 13 | B | 35 | A |
| 14 | A | 36 | A |
| 15 | B | 37 | A |
| 16 | C | 38 | B |
| 17 | A | 39 | A |
| 18 | B | 40 | A |
| 19 | A | 41 | A |
| 20 | A | 42 | A |
| 21 | A | 43 | A |
| 22 | A | 44 | A |
| 23 | A | 45 | A |
| 24 | A | 46 | A |
| 25 | A | 47 | A |

(to be continued)

Table 6 (continued)

| Compound No. | Effect | Compound No. | Effect |
|---|---|---|---|
| 48 | A | 55 | B |
| 49 | A | 56 | C |
| 51 | A | 57 | A |
| 52 | A | 58 | C |
| 54 | A | | |

Test Example 6

Controlling effect on late blight (Phytophthora infestans)

Potted tomato plants at the 3 leaf stage were sprayed with a 200 ppm liquid chemical containing each compound of the present invention as active ingredient. After 24 hours, they were inoculated with a suspension of zoospores of Phytophthora infestans by spraying.

After the inoculation, the plants were placed in a moist chamber at 25°C for 1 day and then a greenhouse for 6 days to cause the disease sufficiently. Thereafter, the percentage of lesion area of each leaf was measured, and in the same manner as in Test Example 5, the controlling degree was calculated and the effect was judged.

The results obtained are shown in Table 7.

Table 7

| Compound No. | Effect | Compound No. | Effect |
|---|---|---|---|
| 1 | C | 25 | B |
| 5 | C | 33 | C |
| 7 | B | 34 | B |
| 8 | C | 35 | B |
| 9 | B | 36 | B |
| 11 | A | 41 | C |
| 13 | C | 43 | B |
| 14 | C | 44 | A |
| 15 | A | 46 | B |
| 17 | C | 52 | C |
| 22 | A | 57 | C |
| 24 | A | 58 | C |

## Claims

1. An aminopyrimidine derivative represented by the general formula (I):

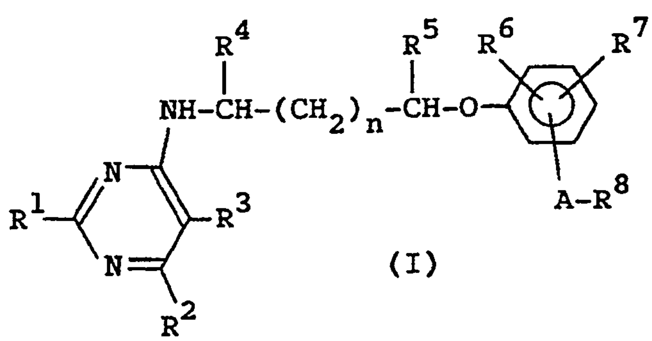

(I)

wherein $R^1$ is a hydrogen atom or a lower alkyl group, $R^2$ and $R^3$, which may be the same or different, are halogen atoms, lower alkyl groups, lower haloalkyl groups, lower alkenyl groups, lower alkynyl groups, cycloalkyl groups, lower alkoxy groups, lower haloalkoxy groups, lower alkylthio groups, or lower haloalkylthio groups, $R^4$ and $R^5$, which may be the same or different, are hydrogen atoms or lower alkyl groups, $R^6$ and $R^7$, which may be the same or different, are hydrogen atoms, halogen atoms, lower alkyl groups, lower haloalkyl groups, lower alkoxy groups, or lower alkylthio groups, A is $-(C(R^9)(R^{10}))_m-$ (in which $R^9$ and $R^{10}$, which may be the same or different, are hydrogen atoms, lower alkyl groups, or lower haloalkyl groups, and m is an integer of 1 to 4), $R^8$ is a phenyl group which may have 1 to 4 substituents which may be the same or different and are selected from the group consisting of halogen atoms, nitro group, cyano group, lower alkyl groups, lower haloalkyl groups, lower alkoxy groups, lower haloalkoxy groups, lower alkylthio

groups, lower haloalkylthio groups, unsubstituted amino group, and substituted amino group having as the substituent(s) one or two lower alkyl groups or acyl groups, which may be the same or different, or $R^8$ is an aromatic fused ring, a naphthyl group, or an oxygen- or sulfur-containing heterocyclic ring which may have 1 to 3 substituents selected from the group consisting of halogen atoms, nitro group, cyano group, lower alkyl groups, lower haloalkyl groups, lower alkoxy groups, lower haloalkoxy groups, lower alkylthio groups, and lower haloalkylthio groups, and n is zero or an integer of 1 to 3.

2. A process for producing an aminopyrimidine derivative represented by the general formula (I):

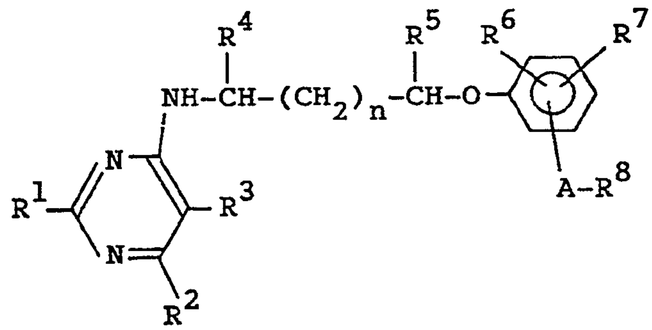

(wherein $R^1$ is a hydrogen atom or a lower alkyl group, $R^2$ and $R^3$, which may be the same or different, are halogen atoms, lower alkyl groups, lower haloalkyl groups, lower alkenyl groups, lower alkynyl groups, cycloalkyl groups, lower alkoxy groups, lower haloalkoxy groups, lower alkylthio groups, or lower haloalkylthio groups, $R^4$ and $R^5$, which may be the same or different, are hydrogen atoms or lower alkyl groups, $R^6$ and $R^7$, which may be the same or different, are hydrogen atoms, halogen atoms, lower alkyl groups, lower haloalkyl groups, lower alkoxy groups, or lower alkylthio groups, A is $-(C(R^9)(R^{10}))_m-$ (in which $R^9$ and $R^{10}$, which may be the same or different, are hydrogen atoms, lower alkyl groups, or lower haloalkyl groups, and m is an integer of 1 to 4), $R^8$ is a phenyl group which may have 1 to 4 substituents which may be the same or different and are selected from the group consisting of halogen atoms, nitro group, cyano group, lower alkyl groups, lower haloalkyl groups, lower alkoxy groups, lower haloalkoxy groups, lower alkylthio groups, lower haloalkylthio groups, unsubstituted amino groups, and substituted amino group having as the substituent(s) one or two lower alkyl groups or acyl groups, which may be the same or different, or $R^8$ is an aromatic fused ring, a naphthyl group, or an oxygen- or sulfur-containing heterocyclic ring which may have 1 to 3 substituents selected from the group consisting of halogen atoms, nitro group, cyano group, lower alkyl groups, lower haloalkyl groups, lower alkoxy groups, lower haloalkoxy groups, lower alkylthio groups, and lower haloalkylthio groups, and n is zero or an integer of 1 to 3) which comprises reacting a halopyrimidine represented by the general formula (II):

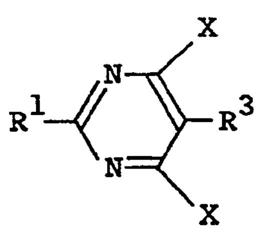

(II)

(wherein $R^1$, $R^2$, and $R^3$ have the same meanings as those defined above, and X is a halogen atom) with an amine represented by the general formula (III):

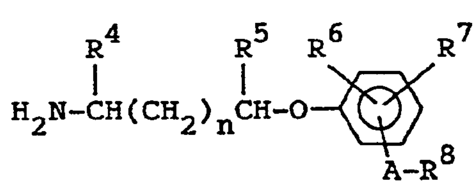

(wherein $R^4$, $R^5$, $R^6$, $R^7$, $R^8$, A and n have the same meanings as those defined above).

3. An insecticidal, acaricidal and/or fungicidal composition comprising a biologically effective amount of an aminopyrimidine derivative represented by the general formula (I):

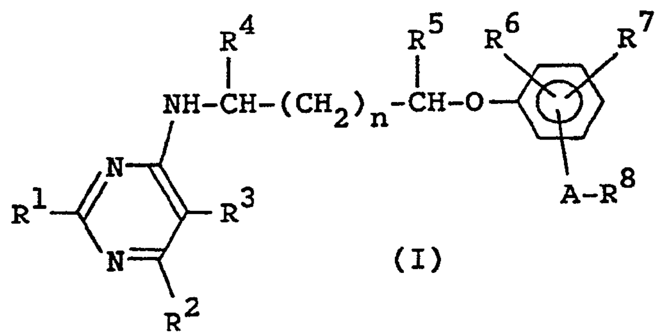

(I)

(wherein $R^1$ is a hydrogen atom or a lower alkyl group, $R^2$ and $R^3$, which may be the same or different, are halogen atoms, lower alkyl groups, lower haloalkyl groups, lower alkenyl groups, lower alkynyl groups, cycloalkyl groups, lower alkoxy groups, lower haloalkoxy groups, lower alkylthio groups, or lower haloalkylthio groups, $R^4$ and $R^5$, which may be the same or different, are hydrogen atoms or lower alkyl groups, $R^6$ and $R^7$, which may be the same or different, are hydrogen atoms, halogen atoms, lower alkyl groups, lower haloalkyl groups, lower alkoxy groups, or lower alkylthio groups, A is $-(C(R^9)(R^{10}))_m-$ (in which $R^9$ and $R^{10}$, which may be the same or different, are hydrogen atoms, lower alkyl groups, or lower haloalkyl groups, and m is an integer of 1 to 4), $R^8$ is a phenyl group which may have 1 to 4 substituents which may be the same or different and are selected from the group consisting of halogen atoms, nitro group, cyano group, lower alkyl groups, lower haloalkyl groups, lower alkoxy groups, lower haloalkoxy groups, lower alkylthio groups, lower haloalkylthio groups, unsubstituted amino group, and substituted amino group having as the substituent(s) one or two lower alkyl groups or acyl groups, which may be the same or different, or $R^8$ is an aromatic fused ring, a naphthyl group, or an oxygen- or sulfur-containing heterocyclic ring which may have 1 to 3 substituents selected from the group consisting of halogen atoms, nitro group, cyano group, lower alkyl groups, lower haloalkyl groups, lower alkoxy groups, lower haloalkoxy groups, lower alkylthio groups, and lower haloalkylthio groups, and n is zero or an integer of 1 to 3) and an agriculturally and horticulturally acceptable carrier.